(19) Europäisches Patentamt
European Patent Office
Office européen des brevets

(11) Publication number: **0 404 671 A1**

(12) **EUROPEAN PATENT APPLICATION**

(21) Application number: **90401736.5**

(22) Date of filing: **15.06.90**

(51) Int. Cl.5: **C07D 213/59, C07D 215/14, C07D 217/14, C07D 237/08, C07D 239/26, C07D 241/12, C07D 209/20, C07D 277/30, C07D 495/04, A61K 31/44, A61K 31/47, //A61K31/495, A61K31/425,A61K31/40, A61K7/06,(C07D495/04,333:00, 221:00)**

(30) Priority: **16.06.89 GB 8913865**

(43) Date of publication of application:
**27.12.90 Bulletin 90/52**

(84) Designated Contracting States:
**AT BE CH DE DK ES FR GB GR IT LI LU NL SE**

(71) Applicant: **RHONE-POULENC SANTE**
**20, avenue Raymond Aron**
**F-92160 Antony(FR)**

(72) Inventor: **Hart, Terance William**
**17 Rowhedge, Hutton**
**Brentwood, Essex(GB)**
Inventor: **Sharp, Brian William**
**12 Strathmore Gardens**
**Hornchurch, Essex(GB)**

(74) Representative: **Pilard, Jacques et al**
**RHONE-POULENC SANTE, Service Brevets,**
**20 Avenue Raymond Aron**
**F-92165 Antony Cédex(FR)**

(54) **Thioformamide derivatives.**

(57) Thioformamide derivatives of the formula:

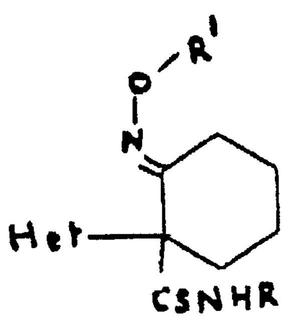

(I)

wherein R represents alkyl of 1 to 4 carbon atoms;
Het represents optionally substituted pyrid-3-yl, isoquinolin-4-yl, tetrahydroquinolin-3-yl, quinolin-3-yl, pyridazin-4-yl, pyrimid-5-yl, thiazol-5-yl, thieno[2,3-b]pyridin-5-yl, pyrazin-2-yl, indol-3-yl or thieno-[3,2-b]-pyridin-6-yl;
and $R^1$ represents a benzyl radical substituted on the ring by two to five fluorine atoms, and salts thereof are therapeutically useful.

EP 0 404 671 A1

Xerox Copy Centre

# THIOFORMAMIDE DERIVATIVES

This invention relates to new therapeutically useful thioformamide derivatives, to processes for their preparation and to pharmaceutical compositions containing them.

The new thioformamide derivatives of the present invention are those compounds of general formula (I) hereinafter depicted, wherein:-

R represents a straight- or branched-chain alkyl radical containing from 1 to 4 carbon atoms;

Het represents an aromatic heterocyclic radical containing one or two nitrogen atoms (optionally substituted by a straight- or branched-chain alkyl or alkoxy radical containing 1 to 4 carbon atoms or by a halogen atom), selected from pyrid-3-yl, isoquinolin-4-yl, tetrahydroquinolin-3-yl, quinolin-3-yl, pyridazin-4-yl, pyrimid-5-yl, thiazol-5-yl, thieno[2,3-b]pyridin-5-yl, pyrazin-2-yl, indol-3-yl, and thieno-[3,2-b]pyridin-6-yl;

and $R^1$ represents a benzyl radical substituted on the ring by two to five fluorine atoms,

and pharmaceutically acceptable salts thereof.

Preferably Het represents pyrid-3-yl. Compounds of general formula (I) which conform to general formula (Ia) are preferred.

In certain cases the substituent R can contribute to stereoisomerism. All such forms are embraced by the present invention.

Particular compounds of the present invention are as follows:-

1. (1S)-anti-N-methyl-2-(2,3,4,5,6-pentafluorobenzyloxyimino-1-(3-pyridyl)cyclohexanecarbothioamide
2. (1S)-anti-N-methyl-2-(3,4-difluorobenzyloxyimino-1-(3-pyridyl)cyclohexanecarbothioamide as well as their enantiomeric and diastereoisomeric and syn forms, where they exist.

The numbers 1 and 2 are allocated to the compounds for easy reference later in the specification, e.g. in the Table and Examples.

The compounds have valuable pharmacological properties, in particular properties which are indicative of utility in the treatment and/or prophylaxis of disorders associated with:-

(1) vascular smooth muscle contraction including hypertension and other cardiovascular disorders such as congestive heart failure, and conditions associated with tissue ischaemia such as angina, peripheral vascular disease and cerebrovascular disease;

(2) respiratory smooth muscle contraction including reversible airways obstruction and asthma;

(3) contraction of smooth muscle of gastrointestinal tract,urinary bladder and uterus,including peptic ulcers, irritable bowel syndrome and diverticular disease; irritable bladder syndrome; and premature labour.

The compounds also have utility in the inhibition of head hair loss associated with male pattern baldness, by topical application.

For example, compounds of general formula (I) were submitted to tests to measure vaso-relaxant, hypotensive and broncho-relaxant activities. The results are collected below in Table 1.

### Vaso-relaxant activity

The test methods used were adapted from those described by Winslow et al [Eur. J. Pharmacol., 131, 219-228 (1986)] and Karaki [J. Pharmacol Methods, 18, 1-21, (1987)] for differentiating vaso-relaxant activity.

Thoracic aorta was removed from rats and transverse strips, denuded of endothelium, were suspended in a bath containing Krebs solution. The tension was recorded and a contraction induced by addition of 20mM $K^+$ (potassium ion) to the bathing solution. The test compound was added to the bath as a solution in increasing cumulative concentration. The concentration in the bathing solution of the test compound which reduced the $K^+$-induced contraction was determined and expressed in nM as the effective concentration ($EC_{90}$)

### Hypotensive activity

The intravenous dose required to reduce the mean blood pressure in an anaesthetised rat was determined and expressed in µg/kg/min as the effective concentration ($EC_{30}$).

### Broncho-relaxant activity

In *in vivo* tests in the anaesthetised guinea pig, performed according to the method of Dixon W.E. and Brodie T.G., J. Physiol 29, 97-173 (1903), the effect on histamine-induced bronchospasm and blood pressure was determined.

A nebulised aerosol generated from an aqueous solution , having a concentration of 200µg/ml was administered for 1 minute to the anaesthetised guinea pigs.

Table 1

| Activity | Parameter | Compound 1 | Compound 2 |
|---|---|---|---|
| Vaso-relaxant | $EC_{90}$ (nM) | 1.0 | 0.3 |
| Hypotensive | $EC_{30}$ (µg/kg/min) | 0.9 | 0.5 |
| Broncho-relaxant | % maximum | 28 | 17 |
| | bronchospasm | (control 72) | (control 73) |
| | B.P. (mmHg) | 40 | 32 |
| | | (control 41) | (control 47) |

The compounds of formula (I) can be prepared by the application or adaptation of known methods, for example as hereinafter identified. By the term "known methods" as used in this specification is meant methods heretofore used or described in the literature.

In the following, the symbols appearing in the formulae are "as hereinbefore defined", unless otherwise indicated.

According to a feature of the present invention, the compounds of general formula (I) may be prepared by the reaction of a compound of general formula (II) with an isothiocyanate of general formula:

R-N=C=S (III)

wherein R represents a straight- or branched-chain alkyl radical containing 1 to 4 carbon atoms.

The reaction is generally carried out in an anhydrous inert solvent such as tetrahydrofuran, dimethylformamide or hexamethylphosphoramide, or a mixture of these solvents, at a temperature from -80 to +50°C, in the presence of an organic base such as potassium tert.-butoxide or an organo-lithium derivative such as butyllithium, or of sodium hydride.

According to a feature of the present invention, the compounds of general formula (I) may be prepared by the reaction of a compound of general formula (IV) with a compounds of general formula:

$NH_2OR^1$ (V)

or with an acid addition salt thereof (preferably the hydrochloride).

The reaction is generally carried out in the presence of an inorganic base, e.g. sodium carbonate or sodium acetate in an inert organic solvent , e.g. ethanol, or an organic base, e.g. pyridine, which may serve as the solvent, in an inert solvent at a temperature from 0 to 120°C.

A stereoselective synthesis may be performed for example as hereinafter described in the Reference Examples in which a mixture of enantiomers of general formula (VIII) is reacted with a chiral auxiliary agent, e.g. (S)-1-amino-2-methoxymethylpyrrolidine, before being reacted with a compound of general formula (III) as hereinbefore described followed by the removal of the chiral auxiliary agent.

According to a feature of the invention, the compounds of general formula (I), wherein R represents a methyl radical may be prepared by reacting methylamine with a dithioester of general formula (VI), wherein $R^3$ represents a straight- or branched-chain alkyl radical containing 1 to 4 carbon atoms, or a benzyl or carboxymethyl radical.

In general, the reaction is carried out with an excess of methylamine, without a solvent or in an organic solvent such as an aromatic hydrocarbon, an ether or an alcohol, of low molecular weight, or a mixture of these solvents, at a temperature from 20 to130°C, optionally under pressure.

It is particularly advantageous for the thiol formed during the reaction to be fixed in the form of a heavy metal salt using a thiol acceptor such as mercuric chloride.

Compounds of general formula (IV) may be prepared from compounds of general formula (VIII) by methods analogous to those used to prepare compounds of general formula (I) from those of general formula (II).

The dithioester of general formula (VI) can be obtained by the reaction of a strong base with a

compound of general formula (II), followed by reacting the resulting product with carbon disulphide and then with a compound of general formula:

$R^3$-Z (VII)

wherein $R^3$ is as hereinbefore defined and Z represents a halogen atom, preferably a chlorine, bromine or iodine atom, or a reactive ester radical, preferably a mesyloxy or tosyloxy radical.

The reaction is generally carried out in an ether such as tetrahydrofuran, to which hexamethylphosphoramide has generally been added, at a temperature from -20 to +50 °C.

It is particularly advantageous to employ potassium tert.-butoxide as the strong base. Alternatively the organo-lithium derivatives described above may be employed.

The thioformamide derivatives of general formula (I) obtained by the aforedescribed processes can be purified by the usual physical methods, in particular crystallisation and chromatography, especially to obtain the appropriate enantiomer using a chiral column.

Compounds of general formula (II) may be prepared by the application of adaptation of known methods for example as hereinafter described in the Reference Examples.

Compounds of general formula (II) may be prepared by the reaction of a compound of general formula (VIII) with a compound of general formula:

$NH_2OR^1$ (V)

or with an acid salt addition salt thereof in a a manner similar to that hereinbefore described for the preparation of the corresponding compounds of general formula (I).

Compounds of general formula (VIII) may be prepared from the corresponding 2-methoxy-1-(Het)-cyclohexanol, by known methods.

Alternatively compounds of general formula (VIII) may be prepared from the corresponding 1[(Het)-bromomethyl]cyclopentanol, again by known methods.

Compounds of general formula (V) may be prepared by known methods.

By the term "pharmaceutically acceptable salts" as used in this specification is meant salts the anions or cations of which are relatively innocuous to the animal organism when used in therapeutic doses so that the beneficial pharmaceutical properties of the parent compounds of general formula (I) capable of forming salts are not vitiated by side-effects ascribable to those anions or cations.

As well as being useful in themselves as active compounds, acid addition salts of the compounds of general formula (I) capable of forming such salts are useful for the purposes of purification of the parent compounds of general formula (I), for example by exploitation of the solubility differences between the salts and the parent compounds, by techniques well known to those skilled in art. The parent compounds of general formula (I) can be regenerated from their acid addition salts by known methods, for example by treatment with an alkali, e.g. aqueous sodium bicarbonate solution or aqueous ammonia solution.

Suitable acid addition salts for use in pharmaceuticals may be selected from salts derived from inorganic acids, for example hydrochlorides, hydrobromides, phosphates, sulphates and nitrates, and organic acids, for example oxalates, lactates, tartrates, acetates, salicylates, citrates, propionates, succinates, fumarates, maleates, methylene-bis-β-hydroxynaphthoates, gentisates and di-p-toluoyltartrates.

As well as being useful in themselves as active compounds, salts of the compounds of general formula (I) capable of forming salts with bases are useful for the purposes of purification of the parent compounds of general formula (I), for example by exploitation of the solubility differences between the salts and the parent compounds, by techniques well known to those skilled in the art.

Suitable salts with bases include alkali metal (e.g. sodium and potassium), alkaline earth metal (e.g. calcium and magnesium), ammonium and amine (e.g. diethanolamine, triethanolamine, octylamine, morpholine and dioctylmethylamine) salts.

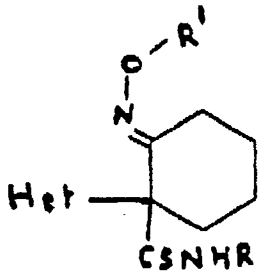
(I)

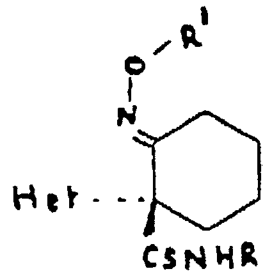
(Ia)

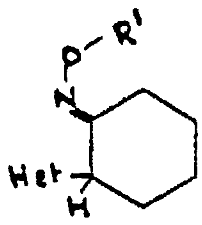
(II)

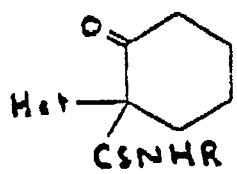
(IV)

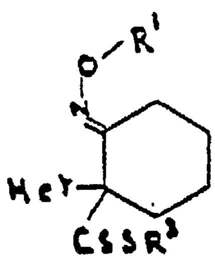
(VI)

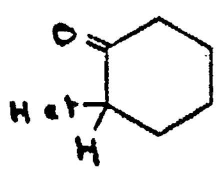
(VIII)

The following Examples and Reference Examples illustrate the preparation of compounds according to the present invention.

Unless stated otherwise, all the spectra were recorded at 200 MHz in deuterochloroform; the chemical shifts are expressed in ppm relative to the tetramethylsilane signal. The abbreviations used in the following text are as follows:

s = singlet
d = doublet
m = multiplet
c = unresolved bands
dd = doublet of doublets
ddd = doublet of doublets of doublets

## EXAMPLE 1

### Compound 1

A mixture of (-)-N-methyl-2-oxo-1-(3-pyridyl)cyclohexanecarbothioamide (5.56g;22.4mmol) and 2,3,4,5,6-pentafluorobenzyloxyamine hydrochloride (7.0g;28mmol) in pyridine (35ml) was heated for 24 hours at 60°C. The mixture was poured into water (175ml) and extracted with ethyl acetate (2x150ml). The combined organic extracts were then washed with hydrochloric acid (0.002N) until the washings attained pH5. The organic layer was then washed successively with saturated aqueous sodium bicarbonate solution (50ml), saturated aqueous sodium chloride solution (50ml) and then dried over magnesium sulphate. Concentration in vacuo afforded a crude oil (8.9g) which was recrystallised from petrol (60-80°C) to give (1S)-anti-N-methyl-2-(2,3,4,5,6-pentafluorobenzyloxyimino)-1-(pyrid-3-yl)cyclohexanecarbothioamide (7.4g), m.p. 114-116°C.
Found:-C, 54.2; H, 3.9; N, 9.5; S, 7.4%
Calculated for $C_{20}H_{18}F_5N_3OS$:-C, 54.2;
H. 4.1; N, 9.5; S, 7.2%.
$[\alpha]_D^{25}$ = -43° (c=1.$CHCl_3$)

## EXAMPLE 2

### Compound 2

A mixture of (-)-N-methyl-2-oxo-1-(3-pyridyl)-cyclohexanecarbothioamide (7.0g;28.2mmol) and 3,4-difluorobenzyloxyamine hydrochloride (8.27g;42.4mmol) in pyridine (100ml) was heated for 24 hours at 60°C. The mixture was poured into water (500ml) and the pH adjusted to 9 using saturated aqueous sodium bicarbonate solution. The mixture was then extracted with ethyl acetate (3x150ml). The combined organic extracts were then washed with hydrochloric acid (0.002N) until the washings attained pH5. The organic layer was then washed successively with saturated aqueous sodium bicarbonate solution (100ml), water (2x100ml) and brine (100ml) and then dried over magnesium sulphate. Concentration in vacuo afforded a crude oil (11.14g) which was recrystallised from petrol (60-80°C) to give (1S)-anti-N-methyl-2-(3,4-difluorobenzyloxyimino)-1-(pyrid-3-yl)cyclohexanecarbothioamide (9.3g), m.p. 79.5-81°C.
Found:-C, 61.6; H, 5.5; N, 10.7; S, 8.6%
Calculated for $C_{20}H_{21}F_2N_3OS$:-C, 61.7;
H, 5.4; N, 10.8; S, 8.2%.
$[\alpha]_D^{25}$ = -67.4° (c=1,$CHCl_3$)

## REFERENCE EXAMPLE 1

A solution of (2S)-anti-2-methoxymethyl-1-[2′-(3-pyridyl)-2′-methylthiocarbamoylcyclohexylideneamino]-pyrrolidine, in 2M aqueous hydrochloric acid (400ml) was stirred at 60°C for 12 hours. The solution was washed with methylene chloride (3x150ml). The aqueous phase was brought to pH 8 with 2M aqueous sodium hydroxide solution. The precipitate was extracted into methylene chloride (500ml + 3x150ml). The combined extracts were washed with water (3x50ml), dried over magnesium sulphate and evaporated. The residual solid was washed with ethyl acetate (50ml) and recrystallised from methanol to give the (S)-isomer of N-methyl-2-oxo-1-(3-pyridyl)-cyclohexanecarbothioamide colourless crystals (12.04g), m.p. 193-194°C,-$[\alpha]^{30}$ -83° ($CHCl_3$).

## REFERENCE EXAMPLE 2

A 2.5M solution of n-butyllithium in hexane (47ml) was added dropwise to a stirred solution of (2S)-2-methoxymethyl-1-[2-(3-pyridyl)-cyclohexylideneamino]pyrrolidine (50:50 mixture of diastereoisomers) (30.73g) in dry tetrahydrofuran (410 ml) at -75° C under argon during 15 minutes, to give a dark red solution. After 30 minutes, a solution of methyl isothiocyanate (8.63g) in tetrahydrofuran (65ml) was added during 10 minutes. The solution was allowed to warm to to 0° C during 45 minutes, maintained at this temperature for 1 hour and then at 20° C for 1 hour, giving a yellow solution. This solution was quenched with saturated aqueous ammonium chloride solution (270ml). The organic layer was separated and the aqueous layer extracted with ether(3x50ml). The combined extracts were washed with brine (2x25ml), dried over magnesium sulphate and evaporated (40° C/0.2mmHg) to give crude (2S)-anti-2-(methoxymethyl)-1-[2′-(3-pyridyl)-2′-methylthiocarbamoylcyclohexylideneamino]pyrrolidine, a semi-crystalline oil (39.81g)

This was the (2'S)-isomer.

## REFERENCE EXAMPLE 3

A mixture of (±)-2-(3-pyridyl)cyclohexanone (20.6g;117.6mmol), (S)-(-)-1-amino-2(methoxymethyl)-pyrrolidine ('SAMP')(15.4g;118mmol) and p-toluenesulphonic acid (316mg) was refluxed in toluene (475ml) for 4 hours. The toluene was removed in vacuo to give an oil which was dissolved in diethyl ether (340ml). The ethereal solution was washed with saturated aqueous sodium bicarbonate solution (2x170ml) and the organic phase dried over magnesium sulphate and concentrated in vacuo to give (2S)-(+)-2-(methoxymethyl)-1-[2-(3-pyridyl)cyclohexylideneamino]pyrrolidine and (2S)-(-)-2-(methoxymethyl)-1-[2-(3-pyridyl)cyclohexylideneamino]pyrrolidine, as a 50:50 mixture of diastereoisomers (30.8g)

Found:-C, 70.6; H, 8.5; N, 14.2%.

Calculated for $C_{17}H_{15}N_3O$:-C, 71.0; H, 8.8; N, 14.6%.

## REFERENCE EXAMPLE 4

A 4:1 mixture of (±)-cis and trans-2-methoxy-1-(3-pyridyl)cyclohexanol (2g, 10 mmol), toluene and phosphorus pentoxide (3.4g, 24 mmol) was heated at reflux for 5 hours. The mixture was then filtered and the precipitate was portioned between 2M sodium hydroxide solution (80 ml) and diethyl ether (25ml). The aqueous layer was extracted with ether (3x25 ml) and the combined organic extracts were dried over sodium sulphate. Concentration in vacuo afforded a crude oil which was purified by flash chromatography to give 2-(3-pyridyl)cyclohexanone (0.79, 4mmol).

## REFERENCE EXAMPLE 5

To a solution of 2.5M n-butyl lithium in hexane (13.2ml, 33 mmol) at -78° C (was added diethyl ether (15 ml) followed by a solution of 3-bromopyridine (4.7g, 30 mmol) in ether (90 ml) over a period of 10 minutes. After 1 hour at -78° C a solution of (±)-2-methoxycyclohexanone (3.84g, 30 mmol) in ether (20 ml) was added dropwise during 10 minutes. After 2 hours at -78° C and 30 minutes at 0° C the reaction mixture was warmed to 20° C and then poured onto ice (150g). The mixture was extracted with ether (2 x 50 ml) and then the combined organic extracts were extracted with 1N hydrochloric acid (50 ml). This aqueous extract was washed with ether (20 ml) and then treated with 2M sodium hydroxide solution (25 ml) and extracted with ether (3 x 100 ml). The organic extracts were combined, washed with brine then dried over anhydrous sodium sulphate. Concentration in vacuo afforded (±)-2-methoxy-1-(3-pyridyl)- cyclohexanol (5.0g, 24 mmol) as a 4:1 mixture of cis and trans isomers; N.M.R. ($CDCl_3$), 1.2 - 2.14 (c), 2.24 - 2.44 (m), 2.90 - 3.28 (c); 3.48 - 3.60 (m), 7.18 - 7.30 (m); 7.78 - 7.96 (m); 8.40 - 8.48 (m); 8.62 - 8.72 (m); 8.78 - 8.82 (m).

## REFERENCE EXAMPLE 6

A solution of (±)-trans-1-[(3-pyridyl)bromomethyl]cyclopentanol (10.24 g, 40 mmol) in anhydrous tetrahydrofuran (500 ml) at 0 °C was treated, dropwise during 30 minutes, with a solution of silver perchlorate (9.9 g, 48 mmol) in anhydrous tetrahydrofuran (50 ml). After 60 minutes at 0 °C the mixture was poured into a mixture of saturated aqueous brine solution (500 ml) and 10% w/v aqueous sodium bicarbonate solution (500 ml). The resulting mixture was filtered and then extracted with ethyl acetate (2 x 500 ml). The combined organic extracts were washed with brine and then dried over sodium sulphate. Concentration in vacuo (30 °C; 14 mmHg) afforded a crude oil which was recrystallised from cyclohexane (120 ml) to give (±)-2-(3-pyridyl)cyclohexanone (6.7 g, 38 mmol), m.p. 78-80 °C. N.M.R. ($CDCl_3$) 1.72-2.12 (m, 4H); 2.12-2.40 (m, 2H); 2.40-2.64 (m, 2H); 3.56-3.72 (dd, 1H); 7.22-7.32 (m, 1H); 7.44-7.54 (ddd, 1H); 8.34-8.42 (dd, 1H); 8.46-8.54 (dd, 1H).

## REFERENCE EXAMPLE 7

A solution of 3-cyclopentylidenemethylpyridine (62.2 g, 0.39 mol) in acetone (600 ml) and water (100 ml) was treated with a solution of concentrated sulphuric acid (18.9 g, 0.19 mol) in water (100 ml) at 5 °C. The ice-cold solution was treated with 1,3-dibromo-5,5-dimethylhydantoin (55 g, 0.19 mol) during 20 minutes. After 3.5 hours at 0 °C the mixture was treated with sodium bicarbonate (33.6 g, 0.4 mol) followed by water (2 l) and then extracted with ethyl acetate (2 x 500 ml). The organic phase was removed and washed with 10% w/v aqueous sodium bicarbonate solution (500 ml) followed by water (200 ml) and brine (200 ml).

The crude extract was dried over sodium sulphate and then filtered through a column of flash silica gel (10 cm x 2.4 cm diameter). After concentration in vacuo (20 °C, 14 mmHg) the dark oil crystallised on standing to give (±)-trans-1-[(3-pyridyl)bromomethyl]cyclopentanol (56 g, 0.22 mol) melting point 92-94 °C.

Calculated for $C_{11}H_{14}BrNO$, C, 51.6; H, 5.5; Br, 31.2; N, 5.5%. Found C, 51.9; H, 5.6; Br, 30.6; N, 5.5%.

N.M.R. ($CDCl_3$); 1.36-2.06 (c, 8H); 2.32-2.46 (broad singlet, 1 H); 5.02 (s, 1H); 7.24-7.34 (ddd, 1H); 8.0-8.1 (ddd, 1H); 8.52-8.56 (dd, 1H); 8.62-8.66 (d, 1H).

## REFERENCE EXAMPLE 8

A suspension of cyclopentyltriphenylphosphonium bromide (226 g, 0.55 mol) in anhydrous tetrahydrofuran (1000 ml) at 2 °C was treated with vigorous stirring under an atmosphere of argon, with potassium t-butoxide (61.7 g, 0.55 mol). The dark red mixture was stirred at 5 °C for 80 minutes and then treated with pyridine-3-carbaldehyde (58.9 g, 0.55 mol) during a period of 20 minutes. The reaction mixture was stirred at 0 °C for 2 hours and then at 20 °C for 18 hours. The tetrahydrofuran was removed in vacuo - (30 °C; 14 mmHg) and the residue extracted with pentane (2 x 500 ml). After treatment with decolourising charcoal (5 g) the mixture was filtered through a plug of flash silica gel (Merck 70-230 mesh (13 cm x 2 cm diameter). The filtrate was concentrated in vacuo (30 °C, 14 mmHg, then 20 °C; 0.01 mmHg) to afford 3-cyclopentylidenemethylpyridine (54 g, 0.34 mol,) as an oil which was used without further purification. N.M.R. ($CDCl_3$) 1.6-1.95 (m, 4H); 2.4-2.65 (m, 4H); 6.26-6.34 (m, 1H); 7.16-7.25 (ddd, 1H); 7.56-7.65 (ddd, 1H); 8.52-8.52 (d, 1H).

The present invention includes within its scope pharmaceutical compositions which comprise a compound of general formula (I) or a pharmaceutically acceptable salt thereof, in association with a pharmaceutically acceptable carrier or coating. In clinical practice the compounds of the present invention may be administered rectally, but are preferably administered parenterally, by inhalation if appropriate, or, more preferably, orally.

Solid compositions for oral administration include compressed tablets, pills, powders and granules. In such solid compositions, one or more of the active compounds is, or are, admixed with at least one inert diluent such as starch, sucrose or lactose. The compositions may also comprise, as is normal practice, additional substances other than inert diluents, e.g. lubricating agents, such as magnesium stearate.

Liquid compositions for oral administration include pharmaceutically acceptable emulsions, solutions, suspensions, syrups and elixirs containing inert diluents commonly used in the art such as water and liquid paraffin. Besides inert diluents such compositions may comprise adjuvants, such as wetting, and suspending agents, and sweetening, flavouring, perfuming and preserving agents. The compositions according to the invention for oral administration also include capsules of absorbable material such as gelatin, containing

one or more of the active substances with or without the addition of diluents or excipients.

Preparations according to the invention for parenteral administration include sterile aqueous, aqueous-organic, and organic solutions, suspensions and emulsions. Examples of organic solvents or suspensing media are propylene glycol, polyethylene glycol, vegetable oils such as olive oil and injectable organic esters such as ethyl oleate. The compositions may also contain adjuvants such as stabilising, preserving, wetting, emulsifying and dispersing agents. They may be sterilized by, for example, filtration through a bacteria-retaining filter, by incorporation in the compositions of sterilizing agents, by irradiation or by heating. They may also be manufactured in the form of sterile solid compositions, which can be dissolved in sterile water or some other sterile injectable medium immediately before use.

Compositions for inhalation may be sterile aqueous solutions which are then nebulised or dry powders formulated in accordance with known methods.

Solid compositions for rectal administration include suppositories formulated in accordance with known methods and containing one or more of the compounds of formula (I) or a pharmaceutically acceptable salt thereof.

The percentage of active ingredient in the compositions of the invention may be varied, it being necessary that it should constitute a proportion such that a suitable dosage shall be obtained. Obviously, several unit dosage forms may be administered at about the same time. The dose employed will be determined by the physician, and depends upon the desired therapeutic effect, the route of administration and the duration of the treatment, and the condition of the patient. In the adult, the doses are generally from 0.001 to 50 mg/kg body weight per day by oral administration. By inhalation, either as a nebulised solution or as a formulated dry powder, the preferred daily dosage is from 0.001 to 5 mg/kg body weight. The compounds may also be applied topically for inhibition of head hair loss associated with male pattern baldness, the preferred daily dosage being from 0.1 to 10 mg/kg body weight applied, for example, in 5ml portions two or three times per day.

The following Example illustrates pharmaceutical compositions according to the present invention.

## COMPOSITION EXAMPLE

No. 2 size gelatin capsules each containing:-

| | |
|---|---|
| (1S)-anti-N-methyl-2-(2,3,4,5,6-pentafluorobenzyloxyimino-1-(3-pyridyl)cyclohexanecarbothioamide | 20mg |
| lactose | 100 mg |
| starch | 60 mg |
| dextrin | 40 mg |
| magnesium stearate | 1 mg |

were prepared in accordance with the usual procedure.

### Claims

1. A thioformamide derivative of the general formula:

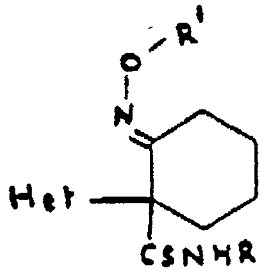

(I)

wherein R represents a straight- or branched-chain alkyl radical containing from 1 to 4 carbon atoms; Het represents an aromatic heterocyclic radical containing 1 or 2 nitrogen atoms (optionally substituted by a straight- or branched-chain alkyl or alkoxy radical containing 1 to 4 carbon atoms or by a halogen atom), selected from pyrid-3-yl, isoquinolin-4-yl, tetrahydroquinolin-3-yl, quinolin-3-yl, pyridazin-4-yl, pyrimid-5-yl, thiazol-5-yl, thieno[2,3-b]pyridin-5-yl, pyrazin-2-yl, indol-3-yl and thieno-(3,2-b]pyridin-6-yl; and $R^1$ represents a benzyl radical substituted on the ring by two to five fluorine atoms, and pharmaceutically acceptable salts thereof.

2. A compound according to claim 1 wherein the thioformamide derivative is of the general formula:

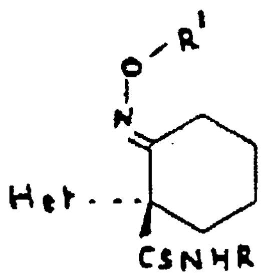

(Ia)

wherein the various symbols are as defined in claim 1.

3. A compound according to claim 1 or 2 in which Het represents pyrid-3-yl.

4. A compound according to claim 1 which is:
(1S)-anti-N-methyl-2-(2,3,4,5,6-pentafluorobenzyloxyiminol-(3-pyridyl)cyclohexanecarbothioamide or (1S)-anti-N-methyl-2-(3,4-difluorobenzyloxyimino-1-(3-pyridyl) cyclohexanecarbothioamide.

5. A process for the preparation of a thioformamide derivative according to claim 1 which comprises the reaction of a compound of the general formula:

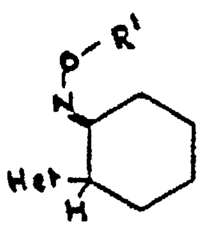

(II)

wherein Het and $R^1$ are as defined in claim 1 with an isothiocyanate of general formula:
R-N=C=S (III)
wherein R is as defined in claim 1.

6. A process for the preparation of a thioformamide derivative according to claim 1 which comprises the reaction of a compound of the general formula:

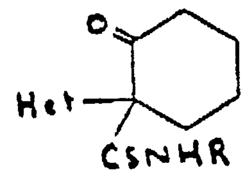

(IV)

wherein Het and R are as hereinbefore defined with a compound of the general formula:

$NH_2OR^1$ (V)

wherein $R^1$ is as defined in claim 1 or with an acid addition salt thereof.

7. A process for the preparation of a thioformamide derivative according to claim 1 wherein R represents a methyl radical which comprises the reaction of methylamine with a dithioester of the general formula:

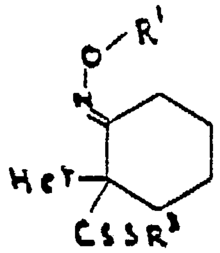

(VI)

wherein $R^3$ represents a straight- or branched-chain alkyl radical containing 1 to 4 carbon atoms or a benzyl or carboxymethyl radical and $R^1$ and Het are as defined in claim 1.

8. A process according to any one of claims 5 to 7 followed by the conversion of a thioformamide derivative of general formula (I) thus obtained into a salt thereof.

9. A pharmaceutical composition which comprises a thioformamide derivative of general formula (I) as defined in claim 1 or a pharmaceutically acceptable salt thereof in association with a pharmaceutically acceptable carrier or coating.

10. A thioformamide derivative of general formula (I) as defined in claim 1 or a pharmaceutically acceptable salt thereof for use as a medicament.

Claims for the following Contracting States: ES, GR

1. A process for the preparation of a thioformamide derivative of the general formula:

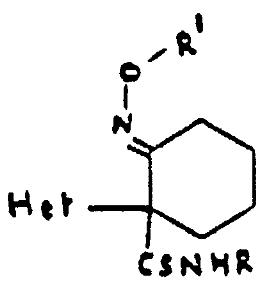

(I)

wherein R represents a straight- or branched-chain alkyl radical containing from 1 to 4 carbon atoms;
Het represents an aromatic heterocyclic radical containing 1 or 2 nitrogen atoms (optionally substituted by a straight- or branched-chain alkyl or alkoxy radical containing 1 to 4 carbon atoms or by a halogen atom), selected from pyrid-3-yl, isoquinolin-4-yl, tetrahydroquinolin-3-yl, quinolin-3-yl, pyridazin-4-yl, pyrimid-5-yl, thiazol-5-yl, thieno[2,3-b]pyridin-5-yl, pyrazin-2-yl, indol-3-yl and thieno-[3,2-b]pyridin-6-yl;
and $R^1$ represents a benzyl radical substituted on the ring by two to five fluorine atoms, and pharmaceutically acceptable salts thereof, which process comprises:
(A) the reaction of a compound of the general formula:

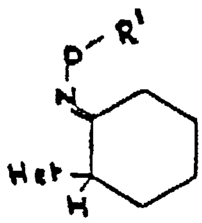

(II)

wherein Het and $R^1$ are as hereinbefore defined with an isothiocyanate of general formula:
R-N=C=S (III)
wherein R is as hereinbefore defined; or
(B) the reaction of a compound of the general formula:

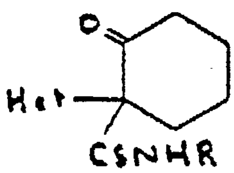

(IV)

wherein Het and R are as hereinbefore defined with a compound of the general formula:
$NH_2OR^1$ (V)
wherein $R^1$ is as hereinbefore defined or with an acid addition salt thereof; or
(C) when R in the thiofornamide derivative of general formula (I) represents a methyl radical, the reaction of methylamine with a dithioester of the general formula:

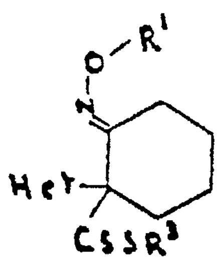

(VI)

wherein $R^3$ represents a straight- or branched-chain alkyl radical containing 1 to 4 carbon atoms or a benzyl or carboxymethyl radical and $R^1$ and Het are as hereinbefore defined;
optionally followed by the conversion of a thioformamide derivative of general formula (I) thus obtained into a salt thereof.

2. A process according to claim 1 in which the thioformamide derivative is of the general formula:

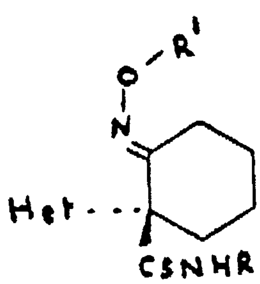

(Ia)

wherein the various symbols are as defined in claim 1.

3. A process according to claim 1 or 2 in which Het represents pyrid-3-yl.

4. A process according to claim 1 in which the thioformamide derivative of general formula (I) is:
(1S)-anti-N-methyl-2-(2,3,4,5,6-pentafluorobenzyloxyimino-1-(3-pyridyl)cyclohexanecarbothioamide or
(1S)-anti-N-methyl-2-(3,4-difluorobenzyloxyimino-1-(3-pyridyl)cyclohexanecarbothioamide.

European Patent Office

# EUROPEAN SEARCH REPORT

Application Number

EP 90 40 1736

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | EP-A-0 321 274 (MAY & BAKER LTD.) * page 3, compounds AM,AW, lines 37,38; example 24; claims 1-5,7-9 * | 1-10 | C 07 D 213/59<br>C 07 D 215/14<br>C 07 D 217/14<br>C 07 D 237/08<br>C 07 D 239/26<br>C 07 D 241/12<br>C 07 D 209/20<br>C 07 D 277/30<br>C 07 D 495/04<br>A 61 K 31/44<br>A 61 K 31/47 //<br>A 61 K 31/495<br>A 61 K 31/425<br>A 61 K 31/40<br>A 61 K 7/06<br>(C 07 D 495/04<br>C 07 D 333:00<br>-/- |
| P,A | EP-A-0 321 273 (MAY & BAKER LTD.) * claims 1-5,7-9 * | 1,5-10 | |

| TECHNICAL FIELDS SEARCHED (Int. Cl.5) |
|---|
| C 07 D 213/00<br>C 07 D 215/00<br>C 07 D 217/00<br>C 07 D 237/00<br>C 07 D 239/00<br>C 07 D 241/00<br>C 07 D 205/00<br>C 07 D 277/00<br>C 07 D 495/00<br>A 61 K 31/00 |

The present search report has been drawn up for all claims

| Place of search | Date of completion of the search | Examiner |
|---|---|---|
| BERLIN | 25-09-1990 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 90 40 1736

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| | | | C 07 D 221:00 ) |
| | | | TECHNICAL FIELDS SEARCHED (Int. Cl.5) |

The present search report has been drawn up for all claims

| Place of search | Date of completion of the search | Examiner |
|---|---|---|
| BERLIN | 25-09-1990 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)